# EUROPÄISCHE PATENTSCHRIFT

(11) **EP 1 277 493 B1**
(45) Veröffentlichungstag und Bekanntmachung des Hinweises auf die Patenterteilung: **21.06.2006**
(21) Anmeldenummer: 02090258.1
(22) Anmeldetag: 16.07.2002
(51) Int. Cl.: A61N 1/372

(54) **Medizinisches Einweggerät**
Medical disposable
Dispositif médical jetable

(30) Priorität: 20.07.2001 DE 10136642
(43) Veröffentlichungstag der Anmeldung: 22.01.2003
(73) Patentinhaber: BIOTRONIK GmbH & Co. KG, 12359 Berlin (DE)
(72) Erfinder: Vitt, Elmar, 20146 Hamburg Rotherbaum (DE); Niewalda, Gregor, 91054 Erlangen (DE); Schaldach, Max, verstorben (DE)
(74) Vertreter: Lindner-Vogt, Karin L.

(56) Entgegenhaltungen:
- EP-A- 0 572 799
- EP-A- 1 192 972
- WO-A-01/35813
- DE-A- 19 903 385
- GB-A- 2 336 214
- US-A- 5 350 407
- US-A- 5 486 200
- US-A- 5 522 856
- US-A- 5 522 897
- US-A- 5 591 217
- US-A- 5 925 069
- US-A- 6 132 086

## Beschreibung

Die vorliegende Erfindung betrifft eine elektrische Therapievorrichtung zur Implantation in einen Körper, eine elektrische Therapievorrichtung nebst eine dazugehörige Verpackung, eine Verpackung für eine elektrische Therapievorrichtung und ein medizinisches Implantat mit einem Gehäuse.

Aus rein technischer Sicht können einige medizinische Einweg-Implantate, wie beispielsweise Herzschrittmacher, Defibrillatoren oder Elektroden, grundsätzlich auch nach einer Explantation dieser Implantate aus dem Patienten wiederverwendet bzw. weiterverwendet werden. Eine Explantation von Implantaten erfolgt beispielsweise bei einer Veränderung der medizinischen Indikation oder nach dem Tod eines Patienten.

Es kommt vor, dass derartige Implantate nach einer Explantation wieder instandgesetzt werden. Dazu wird das Implantat gereinigt, desinfiziert und sterilisiert Eine solche Wiederverwendung erfolgt in der Regel ohne Wissen des Patienten und ohne Billigung durch den Hersteller.

Bei der Entwicklung und Herstellung von medizinischen Produkten für die Einmal-Anwendung sind natürlich zahlreiche Aspekte zu berücksichtigen, die die Sicherheit und die Leistungsfähigkeit derartiger Produkte beeinflussen können. Zu diesen Aspekten gehören beispielsweise die Vorverkeimung des Produktes, das Materialverhalten bei der Anwendung, die Kompatibilität von Produkt, Verpackung und Sterilisationsverfahren, die Haltbarkeitsdauer der Materialien und die Haltbarkeitsdauer der Siegelnähte der Steril-Verpackung. Im Gegensatz zu einem Hersteller verfügen Wiederaufbereiter häufig nicht über diese wichtigen Informationen.

Nach einer Anwendung des Implantates sowie einer anschließenden Wiederaufbereitung durch Reinigung, Desinfektion und Sterilisation können diese medizinischen Produkte Mängel aufweisen. Derartige Mängel können unter anderem im Vorhandensein von Mikrorissen, einer unzureichenden Keimfreiheit, einer unzureichenden Pyrogenfreiheit, in dem Vorhandensein von Partikeln und Endotoxinen, in einer Verschlechterung von Materialeigenschaften, in dem Verbrauch der Batterien, in dem Verlust der elektrischen Sicherheit und in dem Entstehen gefährlicher Stoffe durch Reinigung und Resterilisation begründet sein.

Trotz einer erfolgten Wiederaufbereitung derartiger Implantate bleiben somit bei deren Wiederverwendung einige Risiken erhalten. Eine Beeinträchtigung der Funktionsfähigkeit sowie eine Verringerung der verbleibenden Funktions-Lebensdauer auf Grund der bereits zurückliegenden Benutzungsdauer oder eine Infektion des Patienten kann nicht ausgeschlossen werden. Die dadurch entstehenden Risiken sind zum einen ethisch nicht vertretbar und zum anderen können sie zu Produkthaftungsproblemen führen.

Es ist daher Aufgabe der Erfindung, elektrische Therapievorrichtungen sowie medizinische Implantate vorzusehen, welche eine nicht-autorisierte Wiederverwendung verhindern können.

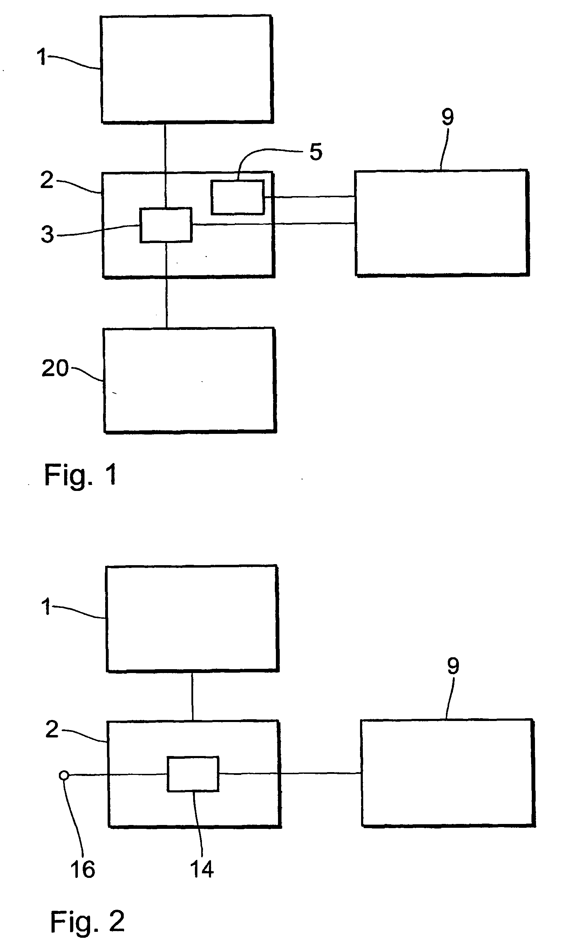

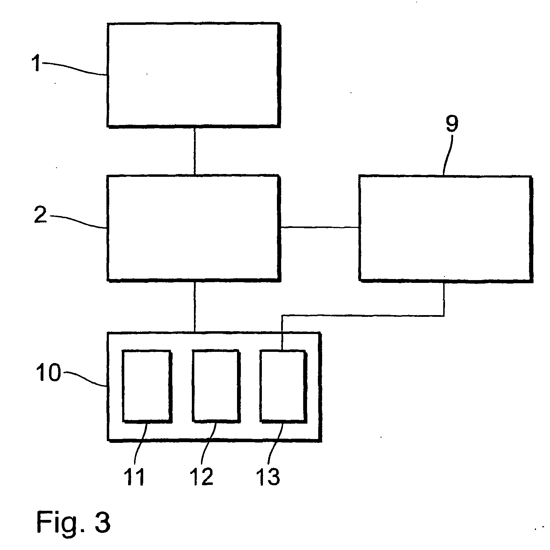

Die Aufgabe der Erfindung wird durch eine elektrische Therapievorrichtung mit den Merkmalen des beigefügten Anspruchs 1 gelöst. Ausführungs beispiele die im Widerspruch zum Gegenstand des Anspruch des Anspruchs 1 stehen sind nicht Gegenstand der Erfindung dies gilt insbesondere für die in den Figuren 1 und 3 gezeigten Vorrichtungen.

Der Erfindung liegt dabei der Gedanke zugrunde, eine elektrische Therapievorrichtung vorzusehen, welche eine Sperr-Auslöseeinheit und eine Wiederverwendungs-Sperreinheit aufweist, wobei die Wiederverwendungs-Sperreinheit die Funktionen der elektrischen Therapievorrichtung während einer Implantation in Abhängigkeit von dem Zustand der Sperr-Auslöseeinheit unterbindet. Die Sperr-Auslöseeinheit wird bei einer Explantation der elektrischen Therapievorrichtung ausgelöst.

Die mit der Erfindung einhergehenden Vorteile liegen insbesondere darin, dass die Wiederverwendungs-Sperreinheit bei der Implantation in einen Körper den Zustand der Sperr-Auslöseeinheit abfragt und anhand dieses Zustandes feststellen kann, ob die elektrische Therapievorrichtung bereits vorher einmal implantiert wurde. Wenn der Zustand der Sperr-Auslöseeinheit darauf hinweist, dass die elektrische Therapievorrichtung bereits einmal implantiert worden ist, unterbricht die Wiederverwendungs-Sperreinheit die Funktionen der elektrischen Therapievorrichtung. Mit anderen Worten, die elektrische Therapievorrichtung lässt sich nicht ein zweites Mal implantieren und somit wiederverwenden. Der Zustand der Sperr-Auslöseeinheit wird bei einer Explantation derart verändert, dass es der Wiederverwendungs-Sperreinheit möglich ist, zu erkennen, dass die elektrische Therapievorrichtung vorab bereits verwendet worden ist.

Der Erfindung liegt hierbei die Überlegung zugrunde, eine elektrische Therapievorrichtung vorzusehen, welche eine Sperr-Auslöseeinheit und eine Wiederverwendungs-Sperreinheit aufweist, wobei die Wiederverwendungs-Sperreinheit die Funktionen der elektrischen Therapievorrichtung während einer Implantation in Abhängigkeit von dem Zustand der Sperr-Auslöseeinheit unterbindet. Die Sperr-Auslöseeinheit wird bei einer Explantation der elektrischen Therapievorrichtung ausgelöst.

Dabei erweist es sich als vorteilhaft, dass der Zustand der Sperr-Auslöseeinheit bei einer Explantation der elektrischen Therapievorrichtung derart gesetzt wird, dass die Wiederverwendungs-Sperreinheit erfassen kann, dass eine Explantation der elektrischen Therapievorrichtung erfolgt ist. Die Wiederverwendungs-Sperreinheit kann die Funktionen der elektrischen Therapievorrichtung sofort unterbinden, sobald sie auf Grund des Zustandes der Sperr-Auslöseeinheit erfasst hat, dass die Therapievorrichtung sich nicht mehr in dem Körper befindet, d. h. eine Explantation erfolgt ist. Bei dieser Variante der Erfindung können die Funktionen der elektrischen Therapievorrichtungen sofort nach Erfassen der Explantation unterbrochen werden, während eine Unterbrechung der Funktionen der elektrischen Therapievorrichtung bei der in Anspruch 1 dargelegten Variante erst bei einer erneuten Implantation der elektrischen Therapievorrichtung in einen Körper erfolgt.

Unter der vorstehend genannten Ausführungsvariante fällt beispielsweise auch ein Herzschrittmacher der mit Inbetriebnahme, nach Anschluss einer Elektrodenleitung, unwiderruflich in einen Zustand versetzt wird der die Unbrauchbarkeit des Herzschrittmachers nach erstmaligen Trennen der Elektrodenleitung von dem Herzschrittmacher direkt zur Folge hat.

Bei dieser Ausführungsvariante muss lediglich das Trennen der Elektrodenleitung von dem Herzschrittmacher detektiert werden, um eine Sperre direkt auszulösen. Ein vorangehender Initialisierungsschritt ist nicht erforderlich.

Unter die genannte Ausführungsvariante fällt beispielsweise auch ein Herzschrittmacher, bei dem mit Anschluss einer Elektrodenleitung ein Bauelement zerstört oder verändert wird, wobei der Herzschrittmacher dann nur noch mit angeschlossener Elektrodenleitung funktionsfähig bleibt. Die Wiederverwendungssperre ist in diesem fall mit Anschluss der Elektrodenleitung Initialisiert. Mit darauffolgendem Trennen der Elektrodenleitung vom Herzschrittmacher wird die Wiederverwendungssperre dann ausgelöst und die Funktion des Herzschrittmachers oder Defibrillators dauerhaft unterbunden. Dies kann dadurch geschehen das die Veränderung oder Zerstörung des entsprechenden Bauteils mit Anschluss der Elektrodenleitung bei darauffolgendem Trennen der Elektrodenleitung die weitere Veränderung der Zerstörung eines Bauteils des Implantates zur Folge hat.

Bei dieser Ausführungsvariante setzt das Unbrauchbarmachen des elektrischen Therapiegerätes somit zunächst eine anfängliche Initialisierung der Wiederverwendungssperre voraus und später eine Auslösen der Wiederverwendungssperre. Die Wiederverwendungssperre muss also zunächst scharf gemacht werden, bevor sie ausgelöst werden kann. Es werden sowohl die Initialisierung der elektrischen Therapievorrichtung bei der Implantation als auch das Auslösen der Wiederverwendungs-Sperre bzw. die ausgelöste Wiederverwendungs-Sperre erfasst, damit die Funktionen der elektrischen Therapievorrichtung unterbunden werden. Mit anderen Worten erfolgt das Unterbinden der Funktionen der elektrischen Therapievorrichtung bei oder im Verlauf der Implantation der elektrischen Therapievorrichtung.

Aus US 5,522,856 und EP 0 572 799 sind jeweils Herzschrittmacher bekannt, die dazu ausgebildet sind, per Impedanzmessung den Anschluss einer Elektrodenleitung zu erfassen, um daraufhin bestimmte Bestandteil der Schrittmachersteuerung zu aktivieren. Eine Wiederverwendungssperre, die eine erneute Inbetriebnahme des Herzschrittmachers nach Entfernen und ggf. Wiederanschließen einer Elektrodenleitung unterbindet, ist aus diesen Veröffentlichungen jedoch nicht bekannt.

Das Dokument US-A-5 486 200 offenbart eine Vorrichrung gemäß Oberbegriff des Anspruchs 1.

Bei einer Ausgestaltung der Erfindung weist die elektrische Therapievorrichtung ferner eine Energieversorgungseinheit auf, welche die Therapievorrichtung mit der notwendigen Energie versorgt. Die Wiederverwendungs-Sperreinheit besitzt hierbei einen Auslöser, welcher dazu geeignet ist die Energieversorgungseinheit, in Abhängigkeit von dem Zustand der Sperr-Auslöseeinheit, von den restlichen Einheiten der elektrischen Therapievorrichtung abzukoppeln, um somit die Energieversorgung der Therapievorrichtung zu unterbrechen.

Durch das Abkoppeln der Energieversorgungseinheit von den restlichen oder zumindest wesentlichen Einheiten der elektrischen Therapievorrichtung, wird sichergestellt, dass die elektrische Therapievorrichtung nicht mehr weiterverwendet werden kann, da sie nicht mehr mit Energie versorgt wird.

Bei einer weiteren Ausgestaltung der Erfindung löst die Wiederverwendungs-Sperreinheit die Sperr-Auslöseeinheit beim Trennen einer Elektrodenleitung von der elektrischen Therapievorrichtung aus.

Bei einer weiteren Ausgestaltung der Erfindung weist die elektrische Therapievorrichtung ein Steuermittel auf, welches die allgemeinen Funktionen der Therapievorrichtung steuert. Die Wiederverwendungs-Sperreinheit ist dabei dazu geeignet, die elektrische Steuerung der Therapievorrichtung durch Anweisungen an das Steuermittel während einer Implantation der elektrischen Therapievorrichtung in Abhängigkeit von dem Zustand der Sperr-Auslöseeinheit zumindest teilweise zu sperren bzw. zu unterbinden. Es wird also der Zustand der Sperr-Auslöseeinheit abgefragt. Zeigt dieser einer Wiederinbetriebnahme an, wird durch das Steuermittel die Funktion des Implantates unterbunden. Hierdurch wird eine elektrische oder elektronische Sperrung der elektrischen Therapievorrichtung ermöglicht, so dass diese nicht weiterverwendet werden kann, da sie bereits einmal in einen Körper implantiert worden ist.

Die Sperr-Auslöseeinheit kann so ausgebildet sein, dass ihr Zustand mit einmaliger Abfrage verändert wird, so dass sich bei einer zweiten Abfrage in einem zweiten Zustand befindet, der anzeigt, dass die Sperr-Auslöseeinheit zuvor bereits abgefragt wurde und somit eine unerwünschte Wiederverwendung versucht wird. Die Zustände der Sperrauslöseeinheit können beispielsweise durch eine einziges Bit in einem Speicher repräsentiert sein, dass im Sinne eines Flags beim ersten Ansprechen des Speichers gesetzt wird und anschließend entweder gar nicht oder nur unter besonderen Bedingungen zurückgesetzt werden kann. Ein geeigneter Speicher wäre beispielsweise ein EPROM.

Somit kann seitens der Wiederverwendungs-Sperreinheit festgestellt werden, wann die Elektrodenleitung von der elektrischen Therapievorrichtung entfernt wird. Anhand dieser Feststellung wird die Sperr-Auslöseeinheit aktiviert und die elektrische Therapievorrichtung wird gesperrt, so dass sie nicht mehr weiterverwendet werden kann.

Weitere mögliche mechanische Wiederverwendungssperren für elektrische oder mechanische Implantate oder Medizingeräte können z.B. Einweg-Rastverbindungen sein. Beispielsweise könne für den Anschluss einer Elektrodenleitung an einen Herzschrittmacher entweder Schrittmacherseitig oder Elektrodenseitig Widerhaken vorgesehen sein, die den Anschluss vorzugsweise auf Seiten des Schrittmachers unwiederbringlich zerstören, indem der schrittmacherseitige Anschluss mechanisch zerstört wird oder nicht entfernbare Überreste eines erforderlichen Verbindungsstücks zwischen Elektrodenleitung und Schrittmacher im Schrittmacherseitigen Anschluss verbleiben, so dass keine Elektrodenleitung mehr an den Schrittmacher angeschlossen werden kann.

Nachstehend wird die Erfindung anhand von Ausführungsbeispielen in Verbindung mit den Zeichnungen näher erläutert. Es zeigen:
- Figur 1: ein Blockschaltbild einer elektrischen Therapievorrichtung gemäß einem ersten Ausführungsbeispiel der Erfindung,
- Figur 2: ein Blockschaltbild einer elektrischen Therapievorrichtung gemäß einem zweiten Ausführungsbeispiel der Erfindung, und
- Figur 3: ein Blockschaltbild einer elektrischen Therapievorrichtung gemäß einem dritten Ausführungsbeispiel der Erfindung,

In Figur 1 ist ein Blockschaltbild einer elektrischen Therapievorrichtung gezeigt. Die elektrische Therapievorrichtung weist ein Steuermittel 1, eine Sperr-Auslöseeinheit 9, eine Wiederverwendungs-Sperreinheit 2 und eine Energieversorgungseinheit 20 auf. Die Wiederverwendungs-Sperreinheit 2 ist jeweils mit dem Steuermittel 1, der Sperr-Auslöseeinheit 9 und der Energieversorgungseinheit 20 verbunden. Die Wiederverwendungs-Sperreinheit 2 weist einen Auslöser 3 und eine erste Empfangseinheit 5 auf. Der Auslöser 3 ist mit dem Steuermittel 1, der Energieversorgungseinheit 20 und der Sperr-Auslöseeinheit 9 verbunden. Die erste Empfangseinheit 5 ist mit der Sperr-Auslöseeinheit 9 verbunden.

Die erste Empfangseinheit 5 ist eine drahtlos aktivierbare Empfangseinheit, wie beispielsweise eine telemetrische Empfangseinheit oder eine magnetisch aktivierbare Empfangseinheit. Die erste Empfangseinheit 5 lässt sich beispielsweise durch ein telemetrisches Signal (bei einer telemetrischen Empfangseinheit) oder durch einen Magneten (bei einer magnetisch aktivierbaren Empfangseinheit) aktivieren. Wenn die erste Empfangseinheit 5 ein entsprechendes telemetrisches oder magnetisches Signal empfängt, aktiviert es die Sperr-Auslöseeinheit 9. Der Auslöser 3 befindet sich zwischen dem Steuermittel 1 und der Energieversorgungseinheit 20 und kann dazu verwendet werden, die Energieversorgungseinheit 20 von dem Rest der elektrischen Therapievorrichtung zu trennen.

Bei der Initialisierung der Therapievorrichtung während ihrer Implantation in einen Körper überprüft die Wiederverwendungs-Sperreinheit 2 den Zustand der Sperr-Auslöseeinheit 9. Wenn die Sperr-Auslöseeinheit 9 zu diesem Zeitpunkt aktiviert ist wird dies an den Auslöser 3 weitergegeben, welcher dann aktiviert wird, und die Verbindung zwischen der Energieversorgungseinheit 20 und dem Steuermittel 1 wird unterbrochen. Die Steuereinheit 1 wird somit nicht mehr mit Energie versorgt und ist folglich abgeschaltet. Die elektrische Therapievorrichtung kann somit nicht weiter verwendet werden.

Der Zustand der Sperr-Auslöseeinheit 9 dient als Indikator dafür, ob die elektrische Therapievorrichtung bereits vorher in einem Körper implantiert worden ist. Bei einer autorisierten Implantation wird die Sperr-Auslöseeinheit 9 nach einer erfolgreichen Implantation der elektrischen Therapievorrichtung mit Hilfe der ersten Empfangseinheit 5 aktiviert. Dies kann entweder telemetrisch oder durch eine magnetische Aktivierung der Empfangseinheit erfolgen.

In Figur 2 ist ein Blockschaltbild einer elektrischen Therapievorrichtung gezeigt. Die Therapievorrichtung weist dabei ein Steuermittel 1, eine Wiederverwendungs-Sperreinheit 2 und eine Sperr-Auslöseeinheit 9 auf. Die elektrische Therapievorrichtung weist ferner einen Anschluss 16 für eine Elektrodenleitung auf. Die Wiederverwendungs-Sperreinheit 2 ist jeweils mit dem Steuermittel 1, der Sperr-Auslöseeinheit 9 sowie dem Anschluss 16 verbunden und weist eine Impedanz-Messeinheit 14 auf. Die Impedanz-Messeinheit 14 ist dabei mit dem Anschluss 16 für die Elektrodenleitung und mit der Sperr-Auslöseeinheit 9 verbunden.

Die Impedanz-Messeinheit 14 erfasst die Impedanz an dem Anschluss 16 und somit die Impedanz einer angeschlossenen Elektrodenleitung. Diese Erfassung kann kontinuierlich oder in diskreten Zeitintervalle erfolgen. In der Impedanz-Messeinheit 14 werden die gemessenen Impedanzwerte mit einem vorab eingegebenen Schwellenwert bzw. Schwellwertbereich verglichen. Wenn die gemessenen Impedanzwerte dabei von diesem Schwellwertbereich abweichen, aktiviert die Impedanz-Messeinheit 14 die Sperr-Auslöseeinheit 9. Die vorab eingegebenen Schwellenwerte bzw. Schwellwertbereiche sind dabei derart gewählt, dass eine Abweichung von diesen Werten lediglich erfolgen kann, wenn die Elektrodenleitung aus dem Anschluss 16 entfernt wird, d. h. wenn die Elektrodenleitung abgetrennt wird. Durch das Messen der Impedanz an dem Anschluss 16 erhält man einen sicheren Indikator dafür, ob Elektrodenleitungen noch an der elektrischen Therapievorrichtung angeschlossen sind oder nicht. Wenn die Elektrodenleitungen nicht mehr an der elektrischen Therapievorrichtung angeschlossen sind, kann dies bedeuten, dass die elektrische Therapievorrichtung aus dem Körper entfernt worden ist.

Bei der Initialisierung der Therapievorrichtung während ihrer Implantation in einen Körper überprüft die Wiederverwendungs-Sperreinheit 2 den Zustand der Sperr-Auslöseeinheit 9. Wenn die Sperr-Auslöseeinheit 9 zu diesem Zeitpunkt aktiviert ist gibt die Wiederverwendungs-Sperreinheit 2 eine Anweisung an das Steuermittel 1 die Steuerung der Funktionen der elektrischen Therapievorrichtung zu sperren. Alternativ dazu kann die Energieversorgung des Steuermittels 1, wie in dem ersten Ausführungsbeispiel zu Fig. 1 beschrieben, gesperrt werden. Dies hat in beiden Fällen zur Folge, dass die elektrische Therapievorrichtung nicht weiter verwendet werden kann.

Alternativ dazu kann die Wiederverwendungs-Sperreinheit 2 beispielsweise die Energieversorgung sofort unterbrechen, sobald die gemessenen lmpedanzwerte zu sehr vom Schwellwertbereich abweichen, d. h. die Elektrodenleitung wurde entfernt.

Figur 3 zeigt ein Blockdiagramm einer elektrischen Therapievorrichtung gemäß dem vierten Ausführungsbeispiel der Erfindung. Die elektrische Therapievorrichtung weist dabei ein Steuermittel 1, eine Wiederverwendungs-Sperreinheit 2, eine Sperr-Auslöseeinheit 9 sowie ein Sperr-Deaktiviermittel 10 auf. Die Wiederverwendungs-Sperreinheit 2 ist jeweils mit dem Steuermittel 1, der Sperr-Auslöseeinheit 9 und dem Sperr-Deaktiviermittel 10 verbunden. Das Sperr-Deaktiviermittel 10 weist eine zweite Empfangseinheit 11, eine Decodiereinheit 12 und eine Verifiziereinheit 13 auf, welche in dieser Reihenfolge nacheinander angeordnet sind. Die zweite Empfangseinheit 11 ist beispielsweise eine telemetrische Empfangseinheit wie sie bereits in dem ersten Ausführungsbeispiel beschrieben worden ist.

Die zweite Empfangseinheit 11 empfängt ein telemetrisches Signal, durch das Daten und Anweisungen übertragen werden. Das empfangende tetemetrische Signal wird in der Decodiereinheit 12 decodiert, so dass die in dem telemetrischen Signal enthaltenen Daten, Anweisungen und Informationen extrahiert werden. Die Verifiziereinheit 13 überprüft diese decodierten Informationen mit einem vorgegebenen Passwort. Wenn die decodierten Informationen oder Daten diesem vorgegebenen Passwort entsprechen, deaktiviert die Verifiziereinheit 13 die Sperr-Auslöseeinheit 9, d. h. der Zustand der Sperr-Auslöseeinheit 9 wird zurückgesetzt. Durch die Verwendung eines Passwortes sowie die Verifizierung dieses Passwortes in der Verifiziereinheit 13 wird sichergestellt, dass die Wiederverwendungs-Sperre in der Sperr-Auslöseeinheit 9 lediglich von autorisierten Personen zurückgesetzt werden kann. Durch die Aufhebung der Wiederverwendungs-Sperre in der Sperr-Auslöseeinheit 9 kann erreicht werden, dass die elektrische Therapievorrichtung bei einer Implantation nicht deaktiviert wird, obwohl diese Implantation möglicherweise bereits die zweite Implantation darstellt.

Die Wiederverwendungs-Sperreinheit 2 kann zum einen sofort nach der Aktivierung der Sperr-Auslöseeinheit 9 oder aber erst bei einer nachfolgenden Implantation die Funktionen der elektrischen Therapievorrichtung sperren bzw. unterbrechen. Weitere Einzelheiten dazu sind bereits vorstehend in Bezug zum ersten und zweiten Ausführungsbeispiel beschrieben.

Die Sperr-Auslöseeinheit 9 kann beispielsweise einen nicht-flüchtigen und nicht-lösbaren Speicher darstellen, in dem zur Aktivierung beispielsweise ein Flag gesetzt wird. Ein derartiger Speicher kann beispielsweise ein EPROM darstellen. Die Wiederverwendungs-Sperre wäre somit aktiviert, wenn dieses Flag gesetzt ist.

Die Sperr-Auslöseeinheit 9 könnte beispielsweise durch einen elektronischen Schalter, wie einen Transistor, einer Sicherung oder einem Relais realisiert werden.

Der Auslöser 3 könnte ebenfalls beispielsweise durch einen elektronischen Schalter, wie einen Transistor, einer Sicherung oder einem Relais realisiert werden.

Sowohl für die Sperr-Auslöseeinheit 9 als auch für den Auslöser 3 wird vorzugsweise eine Sicherung verwendet, welche zur Aktivierung durchbrennbar ist, so dass die Sperr-Auslöseeinheit und der Auslöser 3 nach ihrer Aktivierung nicht mehr aktiviert werden können. Dadurch wäre sichergestellt, dass die elektrische Therapievorrichtung nicht unautorisiert wiederverwendet bzw. aufbereitetet wird.

Unter elektrischen Therapievorrichtungen seien hier Herzschrittmacher Defibrillatoren oder dergleichen verstanden.

## Patentansprüche

1. Elektrische Therapievorrichtung zur Implantation in einen Körper, mit
- einer Sperr-Auslöseeinheit (9), die dazu ausgestaltet ist, bei einer Explantation der elektrischen Therapievorrichtung ausgelöst zu werden, und
- einer Wiederverwendungs-Sperreinheit (2), die dazu ausgestaltet ist, die Funktionen der Therapievorrichtung in Abhängigkeit von dem Zustand der Sperr-Auslöseeinheit (9) zu unterbinden, so dass eine Wiederverwendung der Therapie vorrichtung verbindert wird,
wobei die elektrische Therapievorrichtung ein Herzschrittmacher oder Defibrillator ist,
**dadurch gekennzeichnet, dass**
die Sperr-Auslöseeinheit (9) durch das Trennen einer Elektrodenleitung von dem Herzschrittmacher oder Defibrillator ausgelöst wird und
bei dem die Wiederverwendungs-Sperreinheit (2) die Funktionen des Herzschrittmachers oder Defibrillators nach dem Auslösen der Sperrauslöseeinheit irreversibel unterbindet.

2. Elektrische Therapievorrichtung nach Anspruch 1, **dadurch gekennzeichnet, dass** die Wiederverwendungs-Sperreinheit (2) dazu ausgestaltet ist, die Funktionen der Therapievorrichtung in Abhängigkeit von dem Zustand der Sperr-Auslöseeinheit (9) während einer Implantation der Therapievorrichtung zu unterbinden.

3. Elektrische Therapievorrichtung nach Anspruch 1 oder 2, mit einer Energieversorgungseinheit welche dazu ausgestaltet ist, die Therapievorrichtung mit der notwendigen Energie zu versorgen, und wobei die Wiederverwendungs-Sperreinheit (2) einen Auslöser aufweist, welcher dazu ausgestaltet ist, die Energieversorgungseinheit in Abhängigkeit von dem Zustand der Sperr-Auslöseeinheit (9) abzukoppeln, um die Energieversorgung der Therapievorrichtung zu unterbrechen.

4. Elektrische Therapievorrichtung nach Anspruch 3, wobei die Wiederverwendungs-Sperreinheit (2) dazu ausgestaltet ist, die Sperr-Auslöseeinheit (9) beim Trennen einer Elektrodenleitung von der elektrischen Therapievorrichtung auszulösen.

5. Elektrische Therapievorrichtung nach Anspruch 2, ferner mit einem Steuermittel (1), welches dazu ausgestaltet ist, die allgemeinen Funktionen der Therapievorrichtung zu steuern, wobei die Wiederverwendungs-Sperreinheit (2) dazu ausgestaltet ist, das Steuermittel (1) während einer Implantation in Abhängigkeit von dem Zustand der Sperr-Auslöseeinheit (9) anzuweisen, die elektrische Steuerung der Therapievorrichtung zumindest teilweise zu sperren.

6. Elektrische Therapievorrichtung nach Anspruch 1, 2, 4 oder 5, wobei die Wiederverwendungs-Sperreinheit (2) eine Impedanz-Messeinheit (14) aufweist, die dazu ausgestaltet ist, die Impedanz einer Elektrodenleitung zu bestimmen und die Sperr-Auslöseeinheit (9) auszulösen, wenn die gemessene Impedanz von einem vorgegebenen Wert abweicht.

## Claims

1. An electrical treatment device for implantation in the body, having
- a blocking trigger unit (9), which is implemented for the purpose of being triggered upon an explantation of the electrical treatment device,
- a reuse blocking unit (2), which is implemented for the purpose of suppressing the functions of the treatment device as a function of the state of the blocking trigger unit (9), so that a reuse of the treatment device is prevented,
the electrical treatment device being a pacemaker or defibrillator,
**characterized in that** the blocking trigger unit (9) is triggered by the disconnection of an electrode line from the pacemaker or defibrillator, and
in which the reuse blocking unit (2) irreversibly suppresses the functions of the pacemaker or defibrillator after the triggering of the blocking trigger unit.

2. The electrical treatment device according to Claim 1, **characterized in that** the reuse blocking unit (2) is implemented for the purpose of suppressing the functions of the treatment device as a function of the state of the blocking trigger unit (9) during an implantation of the treatment device.

3. The electrical treatment device according to Claim 1 or 2, having a power supply unit, which is implemented for the purpose of supplying the treatment device with the required power, and wherein the reuse blocking unit (2) has a trigger, which is implemented for the purpose of disconnecting the power supply unit as a function of the state of the blocking trigger unit (9), in order to interrupt the power supply of the treatment device.

4. The electrical treatment device according to Claim 3, wherein the reuse blocking unit (2) is implemented for the purpose of triggering the blocking trigger unit (9) upon disconnection of an electrode line from the electrical treatment device.

5. The electrical treatment device according to Claim 2, also having control means (1), which are implemented for the purpose of controlling the general functions of the treatment device, wherein the reuse blocking unit (2) is implemented for the purpose of instructing the control means (1) to at least partially block the electrical controller of the therapy device during an implantation as a function of the state of the blocking trigger unit (9).

6. The electrical treatment device according to Claim 1, 2, 4, or 5, wherein the reuse blocking unit (2) has an impedance measuring unit (14), which is implemented for the purpose of determining the impedance of an electrode line and triggering the blocking trigger unit (9) if the measured impedance deviates from a predefined value.

## Revendications

1. Dispositif électrique de thérapie à implanter dans un corps, comportant
- une unité de déclenchement de barrage (9) qui est conçue pour se déclencher lors d'une explantation du dispositif électrique de thérapie et
- une unité de barrage à la réutilisation (2) qui est conçue pour inhiber les fonctions du dispositif de thérapie en fonction de l'état de l'unité de déclenchement de barrage (9), de sorte qu'une réutilisation du dispositif de thérapie est empêchée,
le dispositif électrique de thérapie étant un pacemaker cardiaque ou un défibrillateur,
**caractérisé en ce que**
l'unité de déclenchement de barrage (9) est déclenchée par le détachement d'une ligne à électrode du pacemaker cardiaque ou du défibrillateur et
dans lequel l'unité de barrage à la réutilisation (2) inhibe irréversiblement les fonctions du pacemaker cardiaque ou du défibrillateur après le déclenchement de l'unité de déclenchement de barrage (9).

2. Dispositif électrique de thérapie selon la revendication 1, **caractérisé en ce que** l'unité de barrage à la réutilisation (2) est conçue pour inhiber les fonctions du dispositif de thérapie en fonction de l'état de l'unité de déclenchement de barrage (9) pendant une implantation du dispositif de thérapie.

3. Dispositif électrique de thérapie selon la revendication 1 ou 2, comportant une unité d'alimentation en énergie qui est conçue pour alimenter le dispositif de thérapie en énergie nécessaire et dans lequel l'unité de barrage à la réutilisation (2) comporte un déclencheur qui est conçu pour débrancher l'alimentation en énergie en fonction de l'état de l'unité de déclenchement de barrage (9) afin d'interrompre l'alimentation en énergie du dispositif de thérapie.

4. Dispositif électrique de thérapie selon la revendication 3, dans lequel l'unité de barrage à la réutilisation (2) est conçue pour déclencher l'unité de déclenchement de barrage (9) en cas de détachement d'une ligne à électrode du dispositif électrique de thérapie.

5. Dispositif électrique de thérapie selon la revendication 2, comportant en outre un moyen de commande qui est conçu pour contrôler les fonctions générales du dispositif de thérapie, dans lequel l'unité de barrage à la réutilisation (2) est conçue pour ordonner au moyen de commande (1), pendant une implantation, en fonction de l'état de l'unité de déclenchement de barrage (9), de bloquer du moins partiellement la commande électrique du dispositif de thérapie.

6. Dispositif électrique de thérapie selon la revendication 1, 2, 4 ou 5, dans lequel l'unité de barrage à la réutilisation (2) comporte une unité de mesure d'impédance (14) qui est conçue pour déterminer l'impédance d'une ligne à électrode et déclencher l'unité de déclenchement de barrage (9) si l'impédance mesurée diverge d'une valeur prédéfinie.
